# EUROPÄISCHE PATENTSCHRIFT

(11) **EP 1 278 600 B1**
(45) Veröffentlichungstag und Bekanntmachung des Hinweises auf die Patenterteilung: **10.03.2004**
(21) Anmeldenummer: 01933749.2
(22) Anmeldetag: 23.03.2001
(51) Int. Cl.: B01J 23/42, B01J 23/44, B01J 23/48, B01J 23/70, B01J 23/755, B01J 37/02, C07C 5/03, C07C 5/08, C07C 7/163

(54) **SCHALENKATALYSATOREN, VERFAHREN ZU IHRER HERSTELLUNG, SOWIE IHRE VERWENDUNG**
SHELL CATALYSTS, METHOD FOR PRODUCING THE SAME, AND THE USE THEREOF
CATALYSEURS A COQUE, LEUR PROCEDE DE PRODUCTION ET LEUR UTILISATION

(30) Priorität: 28.03.2000 DE 10015250
(43) Veröffentlichungstag der Anmeldung: 29.01.2003
(73) Patentinhaber: BASF AKTIENGESELLSCHAFT, 67056 Ludwigshafen (DE)
(72) Erfinder: ARNOLD, Heiko Dr., Da Chang District Nanjing 210048 (CN); FREIRE ERDBRÜGGER, Cristina, 67251 Freinsheim (DE); HEIDEMANN, Thomas Dr., 68519 Viernheim (DE); MEYER, Gerald, 67067 Ludwigshafen (DE); UNVERRICHT, Signe, 68169 Mannheim (DE); FRENZEL, Andrea Dr., 68535 Edingen-Neckarhausen (DE); WULFF-DÖRING, Joachim, 67105 Schifferstadt (DE); ANSMANN, Andreas, 69168 Wiesloch (DE)
(74) Vertreter: Isenbruck, Günter, Dr.
(86) Internationale Anmeldenummer: PCT/EP2001/003376
(87) Internationale Veröffentlichungsnummer: WO 2001/072415

(56) Entgegenhaltungen:
- EP-A- 0 017 000
- EP-A- 0 547 756
- EP-A- 0 714 700
- WO-A-98/14274
- DE-A- 2 715 094
- US-A- 4 764 498
- US-B1- 6 177 381
- PATENT ABSTRACTS OF JAPAN vol. 004, no. 019 (C-073), 16. Februar 1980 (1980-02-16) & JP 54 157507 A (MITSUBISHI CHEM IND LTD), 12. Dezember 1979 (1979-12-12)

## Beschreibung

Die Erfindung betrifft einen Schalenkatalysator mit einem Kern und mindestens einer, den Kern umgebenden Schale, ein Verfahren zu dessen Herstellung sowie die Verwendung des Schalenkatalysators.

Die für die Hydrierung ungesättigter Kohlenwasserstoffe verwendeten Metallkatalysatoren sind gewöhnlich auf einem homogenen porösen Träger, beispielsweise Calciumcarbonat oder Aktivkohle aufgebracht. Der Katalysator wird dabei in der Weise hergestellt, daß der Träger mit einer Lösung des Metallsalzes getränkt wird. Nach dem Trocknen wird das Metallsalz mit Wasserstoff reduziert und dadurch der Katalysator aktiviert. Solche Katalysatoren weisen eine hohe Umsetzungsrate auf, jedoch ist die Selektivität der Hydrierung oft nicht zufriedenstellend. Dies hat ihre Ursache im Aufbau derartiger Katalysatoren. Durch das Tränken des Trägers mit der Lösung eines Metallsalzes wird nicht immer eine gleichmäßige Verteilung der Aktivkomponente im porösen Träger erreicht. Unter einer Aktivkomponente wird sowohl das katalytisch aktive Metall wie auch dessen Vorläuferverbindung, z.B. ein Metallsalz, verstanden. Im porösen Träger existieren also Bereiche mit einer höheren Konzentration an Aktivkomponente bzw. Bereiche mit einer niedrigeren Konzentration an Aktivkomponente in bezug auf die durchschnittliche Konzentration der Aktivkomponente über das Volumen des gesamten porösen Trägers. Dies führt zu Schwierigkeiten bei der Reaktionsführung, da die Reaktionsbedingungen über das Volumen des Trägers hinweg variieren können. Eine weitere Schwierigkeit besteht darin, daß die Aufenthaltsdauer eines einzelnen Moleküls gegenüber anderen Molekülen im porösen Träger variiert. Dies wird verursacht durch die unterschiedliche Eindringtiefe bzw. Diffusionsgeschwindigkeit der einzelnen Moleküle.

Von einem Hydrierkatalysator wird also gefordert, daß er einerseits eine ausreichende Umsatzrate zeigt und andererseits eine hohe Selektivität. Ferner werden lange Standzeiten zwischen Regenerierungen gefordert sowie inbesondere im Fall von EdelmetallKatalysatoren nach Beendigung der Lebensdauer des Katalysators eine einfache Wiederaufarbeitbarkeit.

In der DE-A-27 15 094 wird ein Katalysator für die selektive Hydrierung von hochungesättigten Kohlenwasserstoffen beschrieben. Dabei wird Palladium auf einen teilchenförmigen porösen Aluminiumoxidträger aufgebracht, wobei das Palladium hauptsächlich in einem Bereich der Katalysatorteilchen verteilt ist, der nicht mehr als 150 µm unter der geometrischen Oberfläche der Teilchen liegt. Die Trägerteilchen können vollständig aus Aluminiumoxid aufgebaut sein. Es ist jedoch auch möglich, daß das Aluminiumoxid als Überzug auf einem anderen Material angeordnet ist. Der Aluminiumoxidträger ist am zweckmäßigsten als Calcinierungsprodukt eines Pseudoböhmits ausgebildet. Der Träger ist über sein gesamtes Volumen porös, ferner werden die Bestandteile des Trägers im Fall eines mehrschichtigen Aufbaus durch chemische Bindungen zusammengehalten, die beispielsweise bei der Calcinierung des Pseudoböhmit zwischen Kern und Schale gebildet werden. Das Palladium wird vorzugsweise durch Naßverfahren auf den Träger aufgebracht, indem der Träger in eine Lösung einer Palladiumverbindung eingetaucht wird oder die Lösung auf den Träger aufgesprüht wird. Das Palladiummetall wird durch Erhitzen oder durch Reduktion mit Wasserstoff freigesetzt. In den Beispielen werden Aluminiumoxidkörner mit einer wässrigen Lösung eines Palladiumsalzes besprüht.

In der EP 0 075 314 werden γ-Aluminiumoxid und Nickel(II)oxid enthaltende bifunktionelle Katalysatoren sowie ihre Herstellung beschrieben. Die Katalysatoren werden zum Cracken von Treibstoffen durch partielle Oxidation verwendet. Als Katalysatoren werden Schalenkatalysatoren mit einem inaktiven Kern und auf diesem in Form von dünnen Schichten vorliegenden katalytisch aktiven Bestandteilen verwendet. Der inerte kompakte Kern der Katalysatoren besteht aus α-Aluminiumoxid und/oder Mullit und/oder gebranntem keramischen Material und/oder Magnesiumoxid und/oder Magnesit. Die Schale des Katalysators besteht aus γ-Aluminiumoxid und Nickel(II)oxid.

Sie wird hergestellt, indem die inerten Trägerteilchen abwechselnd mit einer Nickelsalzlösung besprüht und mit einem aus γ-Aluminiumoxid und Nickel(II)oxid oder aus Aluminiumoxid bestehenden Pulver mit Teilchengrössen von unter 100 µm bestreut werden. Alternativ können die inerten Trägerteilchen auch mit einer aus γ-Aluminiumoxid und Nickel(II)oxid und einer Nickelsalzlösung bereiteten Suspension besprüht werden. Nach dem Aufziehen der Schale wird der Katalysator geglüht.

In der US 4,255,253 wird ein Schalenkatalysator für die Reduzierung des Schwefel-, Stickstoff- oder Metallgehalts von Kohlenwasserstofffraktionen beschrieben, wie sie beispielsweise bei der Erdölverarbeitung anfallen. Der Schalenkatalysator besteht aus einem zumindest in Bereichen porösen Träger mit einem Durchmesser von mindestens 20 µm, der mit einem katalytisch aktiven Material beschichtet ist. Dieses Material besteht aus einer katalytisch aktiven Oxidkomponente und einem Trägermaterial, das eine feste Verbindung mit der Oberfläche des Trägers ausbilden kann. Zur Herstellung des Schalenkatalysators wird der Träger zunächst befeuchtet. Anschliessend wird das pulverförmige katalytisch aktive Material durch schonendes Bewegen des Trägers im Pulver auf die Aussenfläche des Trägers aufgerollt. Als Alternative wird vorgeschlagen, den befeuchteten Träger zunächst mit einem Trägermaterial zu beschichten und nach dem Calcinieren mit einer Lösung von Vorläuferverbindungen der Metalloxide zu imprägnieren.

In der DE 196 07 437 A1 wird ein Verfahren zur Herstellung trägergestützter Metallkatalysatoren mit einem eierschalenförmigen Profil der Verteilung des aktiven Metalls offenbart. Dazu werden metallorganische Verbindungen mit Metallen wie Pd, Ni, Co, Mo, Cu, Pt, Fe, Ag, Ir, Pb, Pi, Sm, V, Zn usw. in einem reinen organischen Lösungsmittel gelöst. Durch Naßimprägnierung und/oder ein Sprühverfahren werden die Metallverbindungen auf die Oberfläche eines Trägers übertragen. Dabei kann das Konzentrationsprofil des Metalls und die Metallbeladung durch Wahl geeigneter Lösungsmittel und/oder Arbeitsbedingungen genau gesteuert werden. Das Trägermaterial des Katalysators ist homogen, d.h. es werden keine weiteren Schichten aus Trägermaterial auf der Aussenseite des Trägerkörpers aufgebracht.

In der EP 0 542 528 wird ein Verfahren zur Hydroisomerisierung von Wachsen beschrieben. Für die Isomerisierung wird ein platinhaltiger Schalenkatalysator verwendet. Für die Herstellung des Katalysators wird auf einen katalytisch inerten Kern eine Schicht aus Böhmit oder Pseudoböhmit aufgebracht, in der das katalytisch aktive Material eingebracht wird. Bei der Calcinierung wandelt sich der Böhmit/Pseudoböhmit in γ-Aluminiumoxid um und bildet eine chemische Bindung zum darunterliegenden Kern aus.

In der EP 0 547 756 A1 wird ein Schalenkatalysator beschrieben, dessen Schale Platin auf fluoriertem Aluminiumoxid enthält. Zur Herstellung des Katalysators wird auf einen Kern aus inertem, katalytisch inaktiven Material, beispielsweise α-Aluminiumoxid, eine Aufschlämmung aus katalytisch aktiven Substanzen und Böhmit oder Pseudoböhmit aufgetragen. Anschliessend werden die Teilchen erhitzt, um den Böhmit/Pseudoböhmit in γ-Aluminiumoxid zu überführen, wobei eine chemische Bindung zwischen Schale und Kern ausgebildet wird.

In der WO 98/37967 wird ein Verfahren zur Herstellung von Schalenkatalysatoren für die katalytische Gasphasenoxidation von aromatischen Kohlenwasserstoffen beschrieben. Dabei ist auf einem Kern aus inertem Material eine Schicht aus katalytisch aktiven Metalloxiden schalenförmig aufgebracht. Als katalytisch aktiver Bestandteil der katalytisch aktiven Masse dient im allgemeinen neben Titandioxid in Form seiner Anatasmodifikation Vanadiumpentoxid. Daneben können in geringen Mengen eine Vielzahl anderer oxidischer Verbindungen enthalten sein, die als Promotoren die Aktivität und Selektivität des Katalysators beeinflussen. Zur Herstellung der Katalysatoren wird aus einer Suspension entsprechender Vorläuferverbindungen durch Sprühtrocknen ein Pulver der katalytisch aktiven Masse hergestellt. Diese wird dann in einer Dragiertrommel auf Formkörper unter Zusatz eines Gemisches aus Wasser und einem organischen Lösungsmittel aufgezogen. Der beschichtete Katalysatorträger wird anschliessend getrocknet.

In der DE 29 09 671 wird ein Verfahren zur Herstellung von Schalenkatalysatoren beschrieben. Die Katalysatoren können zur Oxidation von Acrolein mit Sauerstoff zu Acrylsäure verwendet werden. Zur Herstellung des Schalenkatalysators wird zunächst ein poröser inerter Träger, der den Kern des Schalenkatalysators bildet, mit mindestens 0,1 % seines Gewichts bis 95% seines Wasseraufnahmewertes mit Wasser vorbefeuchtet. Anschliessend werden kontinuierlich mit jeweils konstanter Dosiergeschwindigkeit räumlich getrennt voneinander das katalytisch aktive Material und Wasser auf den stark bewegten Träger aufgegeben. Dabei ist der Wassergehalt der sich bildenden Schale kleiner als der maximale Sättigungsgrad der Schale des katalytisch aktiven Materials. Die Schale des Katalysators enthält neben Molybdän und Vanadium weitere Übergangsmetalle in oxidischer Form.

US 4,764,498 offenbart Schalenkatalysatoren aus einem anorganischen Basismaterial und einer porösen Siliciumdioxid enthaltenden Schicht, die eine katalytisch aktive Substanz enthalten kann. Das anorganische Basismaterial kann nicht-porös, leicht-porös oder starkporös sein und ist inert. Es werden eine Reihe katalytisch aktiver Metalle aufgezählt, die auch Metalle der 10. und 11. Gruppe des Periodensystems umfassen. Die Schalenkatalysatoren können unter anderem zur Hydrierung von ungesättigten Kohlenwasserstoffen eingesetzt werden.

In der EP 0 714 700 A2 wird ein Verfahren zur Herstellung eines Katalysators, bestehend aus einem Kern und einer auf der Oberfläche des Kerns aufgebrachten katalytisch aktiven Oxidmasse beschrieben. Dazu wird ein Trägerkörper, der den Kern des Schalenkatalysators bildet, mit einer wässrigen Lösung einer bei Normaldruck oberhalb von 100°C siedenden organischen Substanz als Haftflüssigkeit zunächst befeuchtet und danach durch Inkontaktbringen mit trockener, feinteiliger aktiver Oxidmasse an der Oberfläche des befeuchteten Trägerkörpers eine Schicht aktiver Oxidmasse angeheftet. Die Haftflüssigkeit wird anschliessend aus dem mit aktiver Oxidmasse beschichteten, befeuchteten Trägerkörper entfernt. Die katalytisch aktive Oxidmasse enthält als Metalle Molybdän und Vanadium sowie weitere Metalle.

Aufgabe der Erfindung ist es, einen Katalysator für die Gasphasenhydrierung von ungesättigten Kohlenwasserstoffen zur Verfügung zu stellen, der eine erhöhte Aktivität aufweist.

Diese Aufgabe wird gelöst durch ein Verfahren zur Herstellung eines Schalenkatalysators mit einem Kern und mindestens einer, den Kern umgebenden Schale, wobei der Kern aus einem inerten Trägermaterial aufgebaut ist, die mindestens eine Schale aus einer porösen Trägersubstanz aufgebaut ist, wobei die Schale physikalisch an den Kern angeheftet ist, und in der mindestens einen Schale zumindest ein katalytisch aktives Metall, das aus der Gruppe ausgewählt ist, die von den Metallen der 10. und 11. Gruppe des Periodensystems der Elemente gebildet ist, oder eine Vorstufe des katalytisch aktiven Metalls in gleichmäßig fein verteilter Form enthalten ist, umfassend die Schritte:
a) Bereitstellen eines den Kern des Schalenkatalysators bildenden Trägers aus einem inerten Trägermaterial
b) Bestreuen des Trägers mit einer oxidischen, pulverförmigen Trägersubstanz, wobei der Träger bewegt wird, unter Ausbildung einer Schale;
c) Besprühen des Trägers mit einem flüssigen Bindemittel, bestehend aus einer wässrigen Lösung einer organischen Verbindung, die bei Normaldruck einen Siedepunkt oder einen Sublimationspunkt von mehr als 100°C aufweist;
d) Einbringen eines katalytisch aktiven Metalls, das ausgewählt ist aus der Gruppe, die gebildet ist von den Metallen der 10, und 11. Gruppe des Periodensystems der Elemente oder einer Vorläuferverbindung dieser Metalle;
e) Verdampfen flüchtiger Bestandteile;
f) gegebenenfalls Aktivieren der Vorläuferverbindung, wobei die Schritte b, c und d gleichzeitig oder in beliebiger Reihenfolge nacheinander sowie gegebenenfalls auch mehrmals ausgeführt werden können,
dadurch gekennzeichnet, dass das katalytisch aktive Metall oder die Vorläuferverbindung im Bindemittel gelöst oder suspendiert und während des Besprühens des Trägers mit dem Bindemittel in die Schale des Schalenkatalysators eingebracht oder eine Lösung oder Suspension des katalytisch aktiven Metalls oder der Vorläuferverbindung getrennt auf den Träger aufgesprüht wird, wobei dies während des Aufbaus der Schale oder danach erfolgen kann.

Gelöst wird die Aufgabe auch durch den so hergestellten Schalenkatalysator selbst.

Mit dem erfindungsgemäßen Schalenkatalysator wird eine hohe Selektivität von Hydrierungen in der Gasphase erreicht. Durch die Schale können definierte und gleichmäßige Reaktionsbedingungen geschaffen werden. Da der Kern keine Aktivkomponente enthält, erfolgt eine katalytische Umsetzung nur in der Schale des Schalenkatalysators, deren Schichtdicke und Zusammensetzung definiert gewählt werden kann. Die Diffusionswege der Reaktanden in der Schale sind kurz und für alle Moleküle annähernd gleich. Zwischen Kern und diesen umgebender Schale besteht nur eine physikalische Verbindung, die z.B. durch Schrumpfen der Schale erreicht wird. Es werden keine chemischen Bindungen zwischen Kern und Schale ausgebildet. Die Adhäsion zwischen den einzelnen Partikeln und zwischen Schale und Kern ist jedoch ausreichend, um einen stabilen Aufbau der Katalysatorteilchen zu gewährleisten. Nach Beendigung der Lebensdauer des Schalenkatalysators ist eine leichte Aufarbeitung durch mechanische Trennung von Schale und Kern möglich.
Das katalytisch aktive Metall kann entweder direkt in feinverteilter Form in der Schale vorliegen, oder es wird unter Hydrierbedingungen aus einer Vorstufe des katalytisch aktiven Metalls gebildet, die beispielsweise durch Aufsprühen einer Lösung gleichmäßig in der Schale verteilt wurde.

Als Materialien für den Kern kommen insbesondere Aluminiumoxid, Siliciumdioxid, Silicate wie Ton, Kaolin, Steatit, Bims, Aluminiumsilicat und Magnesiumsilicat, Siliciumcarbid, Zirkondioxid und Thoriumdioxid in Betracht.
Der Kern kann aus einem porösen Trägermaterial aufgebaut sein.
Vorzugsweise beträgt jedoch das Gesamtvolumen der Poren bezogen auf das Volumen des Trägermaterials weniger als 1 Vol.-%. Prinzipiell kommen für den Kern beliebige Geometrien in Betracht. Vorzugsweise werden jedoch Kugeln oder Zylinder, insbesondere Hohlzylinder, als Kern verwendet. Ihre Längsausdehnung beträgt in der Regel 1 bis 10 mm.

Werden Zylinder als Kern verwendet, so beträgt deren Länge vorzugsweise 2 bis 10 mm und ihr Aussendurchmesser bevorzugt 4 bis 10 mm. Im Fall von Ringen liegt die Wanddicke darüber hinaus üblicherweise bei 1 bis 4 mm. Besonders bevorzugte ringförmige Kerne besitzen eine Länge von 3 bis 6 mm, einen Aussendurchmesser von 4 bis 8 mm und eine Wanddicke von 1 bis 2 mm. Ganz besonders bevorzugt sind Ringe der Geometrie 7mm x 3 mm x 4 mm (Aussendurchmesser x Länge x Innendurchmesser).

Mit Vorteil ist die Oberfläche des Kerns rauh, da eine erhöhte Oberflächenrauhigkeit in der Regel eine erhöhe Haftfestigkeit der aufgebrachten Schale bedingt. Vorzugsweise liegt die Oberflächenrauhigkeit R_{z} des Kerns im Bereich von 40 bis 200 µm, vorzugsweise 40 bis 100 µm (bestimmt gemäß DIN 4768 Blatt 1 mit einem "Hommel Tester für DIN-ISO Oberflächenmeßgrößen" der Fa. Hommelwerke). Die auf den Kern aufgebrachte Trägersubstanz weist eine hohe Porosität auf. Als Materialien für die Schale können dieselben Materialien verwendet werden wie für den Kern. Besonders bevorzugt sind Aluminiumoxid, Zircondioxid, Titandioxid und Siliciumdioxid. Die Trägersubstanz der Schale ist im allgemeinen chemisch inert, greift also in den Ablauf der Gasphasenhydrierung, die durch die erfindungsgemäßen Schalenkatalysatoren katalysiert wird, im wesentlichen nicht ein. In Abhängigkeit von der katalysierten Reaktion ist es jedoch auch möglich, saure oder basische Trägersubstanzen zu verwenden. In der Schale sind die Edelmetallkatalysatoren in feinverteilter Form eingebracht.

Die Metalle können auch in Form einer Vorläuferverbindung in gleichmäßig verteilter Form in der Schale des Schalenkatalysators enthalten sein. Diese vorläufigen Verbindungen können unter den Hydrierbedingungen der katalysierten Reaktion in die aktive Form der reinen Metalle überführt werden. Geeignete Vorläuferverbindungen der katalytischen Metalle sind die entsprechenden Metalloxide oder wasserlöslichen Metallsalze. Bevorzugt verwendet werden die Chloride, Nitrate, C₁-C₁₀ Carboxylate, Carbonate, Hydrogencarbonate, Sulfate, Hydrogensulfate oder Phosphate. Der Vorteil der Wasserlöslichkeit der Salze beruht darin, daß die Verbindungen in flüssiger Phase sehr einfach durch Aufsprühen gleichmässig in das Schalenmaterial eingebracht werden können.

Um den Kern des Schalenkatalysators können mehrere übereinander angeordnete Schalen vorgesehen sein. Die Schalen können unterschiedliche katalytisch aktive Metalle enthalten. Es kann auch die Konzentration des katalytisch aktiven Metalls in den verschiedenen Schalen unterschiedlich sein. Auf diese Weise ist eine weitere Verbesserung der Selektivität der Hydrierreaktion möglich.

Die Schichtdicke der erfindungsgemäß auf dem Kern aufgebrachten Schale liegt zweckmäßigerweise bei 1 bis 1000 µm. Bevorzugt sind, insbesondere bei ringförmigen Kernen, Schichtdicken zwischen 10 bis 500 µm, insbesondere zwischen 50 und 300 µm.

Erfindungsgemäß werden die Schalenkatalysatoren mit einem Verfahren hergestellt, umfassend die Schritte:
a) Bereitstellen eines den Kern des Schalenkatalysators bildenden Trägers aus einem inerten Trägermaterial;
b) Bestreuen des Trägers mit einer oxidischen, pulverförmigen Trägersubstanz, wobei der Träger bewegt wird, unter Ausbildung einer Schale;
c) Besprühen des Trägers mit einem Bindemittel, bestehend aus einer wässrigen Lösung einer organischen Verbindung, die bei Normaldruck einen Siedepunkt von mehr als 100°C aufweist;
d) Einbringen eines katalytisch aktiven Metalls, das ausgewählt ist aus der Gruppe, die gebildet ist von den Metallen der 10. und 11. Gruppe des Periodensystems der Elemente oder einer Vorläuferverbindung dieser Metalle;
e) Verdampfen flüchtiger Bestandteile;
f) gegebenenfalls Aktivieren der Vorläuferverbindung der Metalle, wobei die Schritte b), c) und d) gleichzeitig oder in beliebiger Reihenfolge nacheinander sowie gegebenenfalls auch mehrmals ausgeführt werden können.

Die Beschichtung wird im allgemeinen in der Weise durchgeführt, daß die zu beschichtenden Kerne in einen vorzugsweise geneigten (der Neigungswinkel beträgt in der Regel 30 bis 90°) rotierenden Drehbehälter (z.B. Drehteller oder Dragierkessel) gefüllt werden. Der rotierende Drehbehälter führt die insbesondere kugelförmigen oder zylindrischen, insbesondere bevorzugt hohlzylindrischen Kerne unter zwei in bestimmtem Abstand aufeinander folgend angeordneten Dosiervorrichtungen hindurch. Die erste der beiden Dosiervorrichtungen entspricht zweckmässig einer Düse, durch die die im rotierenden Drehteller rollenden Kerne mit dem erfindungsgemäß zu verwendenden flüssigen Bindemittel besprüht und kontrolliert befeuchtet werden. Die zweite Dosiervorrichtung befindet sich ausserhalb des Zerstäubungskegels des eingesprühten flüssigen Bindemittels und dient dazu, die feinteilige, die Schale bildende Trägersubstanz zuzuführen (z.B. über eine Schüttelrinne). Die kontrolliert befeuchteten Kerne nehmen die zugeführte Trägersubstanz auf, die sich durch die rollende Bewegung auf der äusseren Oberfläche des zylinder- oder kugelförmigen Kerns zu einer zusammenhängenden Schale verdichtet.

Bei Bedarf durchläuft der so grundbeschichtete Kern im Verlauf der darauffolgenden Umdrehung wiederum die Sprühdüse, wird dabei kontrolliert befeuchtet, um im Verlauf einer Weiterbewegung eine weitere Schicht feinteiliger Trägersubstanz aufnehmen zu können usw. (eine Zwischentrocknung ist in der Regel nicht erforderlich). In diesem Fall bildet der Kern mit den bereits ausgebildeten Schalen den Träger für die aufzubringende Schale.

Bei der Herstellung des Schalenkatalysators wird zweckmäßigerweise zunächst durch Bestreuen des Kerns mit einer kleinen Menge an trockener oxidischer pulverförmiger Trägersubstanz eine dünne Schale ausgebildet. Diese wird anschliessend durch Besprühen mit Bindemittel fixiert. Im folgenden wird durch weiteres Bestreuen mit oxidischer pulverförmiger Trägersubstanz die Schale bis zur gewünschten Schichtdicke aufgebaut.

Die Feinheit der auf die Oberfläche des Kerns aufzubringenden oxidischen pulverförmigen Trägersubstanz wird an die gewünschte Schalendicke angepasst. Für den Vorzugsbereich einer Schalendicke von 10 bis 500 µm eignen sich insbesondere solche pulverförmigen Trägersubstanzen, von denen 50 % der Pulverpartikel ein Sieb der Maschenweite 1 bis 10 µm passieren und deren Anteil an Partikeln mit einer Längstausdehnung oberhalb von 50 µm weniger als 1% beträgt. In der Regel entspricht die Verteilung der Längstausdehnug der Pulverpartikel herstellungsbedingt einer Gaußverteilung.

Als organische Komponente des flüssigen Bindemittels eignen sich insbesondere ein- und mehrwertige organische Alkohole wie Ethylenglycol, 1,4-Butandiol, 1,6-Hexandiol oder Glycerin, ein- oder mehrwertige organische Carbonsäuren wie Propionsäure, Oxalsäure, Malonsäure, Glutarsäure oder Maleinsäure, Aminoalkohole wie Ethanolamin oder Diethanolamin, ein- oder mehrwertige organische Amide wie Formamid, Monosaccharide oder Oligosaccharide wie Glucose, Fructose, Saccharose oder Lactose. Das Bindemittel besteht bevorzugt aus einer Lösung aus 20 bis 90 Gew.-% Wasser und 10 bis 80 Gew.-% einer in Wasser gelösten organischen Verbindung, deren Siedepunkt oder Sublimationstemperatur bei Normaldruck (1 Atmosphäre) oberhalb von 100 °C liegt. Der organische Anteil an dem zu verwendenden flüssigen Bindemittel beträgt vorzugsweise 10 bis 50 und besonders bevorzugt 20 bis 30 Gew.-%.

Wesentlich für die Herstellung des Schalenkatalysators ist, daß die Befeuchtung mit dem Bindemittel in kontrollierter Weise vorgenommen wird. Die Oberfläche des Kerns bzw. bereits ausgebildeter Schalen wird zweckmäßig so befeuchtet, daß diese zwar flüssiges Bindemittel adsorbiert aufweist, aber auf der Oberfläche keine Flüssigphase als solche visuell in Erscheinung tritt. Ist die Oberfläche zu feucht, agglomeriert die pulverförmige Trägersubstanz zu getrennten Agglomeraten, anstatt auf die Oberfläche aufzuziehen.

Das katalytisch aktive Metall, das ausgewählt ist aus der Gruppe, die gebildet ist von Metallen der 10. und 11. Gruppe des Periodensystems der Elemente, insbesondere Platin, Palladium und Silber oder einer Vorläuferverbindung der Metalle, kann auf verschiedene Weise erfolgen.

Nach einer Ausführungsform ist das katalytisch aktive Metall oder die Vorläuferverbindung im Bindemittel gelöst oder suspendiert. Beim Aufbau der Schale wird das katalytisch aktive Metall oder die Vorläuferverbindung dann während des Besprühens des Trägers mit dem Bindemittel in die Schale des Schalenkatalysators eingearbeitet.

Bei einer weiteren Ausführungsform des Verfahrens wird eine Lösung oder Suspension des katalytischen aktiven Metalls oder der Vorläuferverbindung durch eine getrennte Düse auf den Träger aufgesprüht, wobei dies während des Aufbaus der Schale erfolgen kann oder auch erst nach Aufbau der Schale.

Nach Aufbau der Schale werden abschliessend die flüchtigen Bestandteile in kontrollierter Weise, z.B. durch Verdampfen und/oder Sublimieren, entfernt. Im einfachsten Fall kann dies durch Einwirkung heißer Gase entsprechender Temperatur (häufig 50 bis 150°C) erfolgen. Durch Einwirkung heißer Gase kann aber auch nur eine Vortrocknung bewirkt werden. Die Endtrockung kann dann beispielsweise in einem Trockenofen beliebiger Art (z.B. Bandtrockner) erfolgen. Die einwirkende Temperatur sollte dabei so gewählt werden, daß keine wesentliche Veränderung der Porosität der Schale eintritt.

Ein besonderer Vorzug des erfindungsgemäßen Verfahrens besteht darin, daß in einem Arbeitsgang Schalenkatalysatoren mit zwei oder mehr schichtförmig übereinander angeordneten Schalen unterschiedlicher Zusammensetzung hergestellt werden können. Das erfindungsgemäße Verfahren bewirkt dabei sowohl eine gute Haftung der aufeinanderfolgenden Schichten aneinander, als auch der untersten Schicht auf der Oberfläche des Kerns. Dies gilt auch im Fall von ringförmigen Kernen.

Bei den erfindungsgemäßen Verfahren ist im allgemeinen keine Behandlung bei erhöhter Temperatur (Calcinieren) erforderlich, die eine Verbindung der Körner der pulverfömigen Trägersubstanz durch Anschmelzen bewirkt. Für die Erhöhung der Stabilität des Schalenkatalysators kann es jedoch vorteilhaft sein, daß der Schalenkatalysator nach dem Verdampfen flüchtiger Bestandteile bei einer Temperatur von 200 bis 600°C calciniert wird. Die Calcinierung erfolgt bei vergleichsweise niederer Temperatur.

Die erfindungsgemäßen Schalenkatalysatoren zeigen eine sehr gute Selektivität bei Hydrierreaktionen. Die erfindungsgemäßen Schalenkatalysatoren werden daher bevorzugt für die Gasphasenhydrierung von Kohlenwasserstofffraktionen, vorzugsweise C₂-C₄ Fraktionen verwendet.

Die Erfindung wird im weiteren anhand von Beispielen genauer erläutert.

### Beispiele

### Beispiel 1: Herstellung des Vergleichskatalysators 1

Ein Aluminiumoxid-Träger in Strangform mit einer BET-Oberfläche von 8. m²/g wurde durch Besprühen mit einer salpetersauren, wässrigen Lösung von bezogen auf die eingesetzte Trägermasse 0,045 Gew.-% Silber in Form von Silbernitrat und von bezogen auf die eingesetzte Trägermasse 0,025 Gew.-% Palladium in Form von Palladiumnitrat bei Raumtemperatur imprägniert. Dabei wurden als Lüsungsvolumen 90 % der Wasseraufnahme des Trägers eingesetzt. Der Kontakt wurde bei 80 °C getrocknet und anschließend bei 400 °C calciniert. Lichtmikroskopische Aufnahmen zeigen die Ausbildung einer ca. 250 - 300 µm breiten Aktivkomponenten-Zone im Randbereich der Stränge.

### Beispiel 2: Erfindungsgemäßer Katalysator

Ein erfindungsgemäßer Katalysator 1 wurde hergestellt, indem 500 g unporöse Steatit-Kugeln, die einen Durchmesser von 2,5 bis 3 mm aufwiesen, mit 100 g Versal® (Aluminiumoxid der Fa. La Roche, 5 h bei 1050 °C calciniert, BET-Oberfläche 40 m²/g) durch parallele Zugabe einer Lösung aus Haftvermittler (Glycerin), bezogen auf die eingesetzte Versalmenge 0,045 % Silber eingesetzt als Silbernitrat und bezogen auf die eingesetzte Versalmenge 0,0925% Palladium eingesetzt als salpetersaure Palladiumnitratlösung beschichtet, getrocknet und bei 300 °C calciniert. Durch lichtmikroskopische Messung wurde die Dicke der erhaltenen Schale zu max. 200 µm bestimmt.

### Beispiel 3: Vergleichskatalysator 2

Ein erfindungsgemäBer Katalysator wurde hergestellt, indem Versal® (6 h bei 1100 °C calciniert, BET-Oberfläche 55 m²/g) mit 0,045 % Silber eingesetzt als Silbernitrat und 0,0925 % Palladium eingesetzt als salpetersaure Palladiumnitratlösung getränkt, getrocknet und bei 400 °C calciniert wurde. 500 g unporöse Steatit-Kugeln, die einen Durchmesser von 2,5 bis 3 mm aufwiesen, wurden mit 80 g des mit Silber und Palladium getränkten Versals® unter Zugabe einer wässrigen Lösung aus Haftvermittler (Glycerin) beschichtet, getrocknet und bei 300 °C calciniert.

Die Eigenschaften der in den Beispielen 1 bis 3 beschriebenen Katalysatoren wurden in einer drucklosen Laborapparatur überprüft.

Ein Vorgemisch von 99 Vol.-% Ethylen und 1 Vol.-% Acetylen wurde in einem Festbettreaktor über 66 ml des jeweiligen Katalysators geleitet, wobei das Verhältnis des zum Vorgemisch zugesetzten Wasserstoffs zu Acetylen 1,8:1 und die GHSV 3000 1/h betrug.

Folgende Temperaturen wurden unter Erhalt der jeweiligen Selektivitäten zum Wertprodukt Ethylen für 90 %igen Umsatz des Acetylens benötigt:

| **Katalysator** | **Temperatur [°C]** | **Selektivität zu Ethylen [%]** |
|---|---|---|
| Vergleichskatalysator 1 | 69 | 25 |
| Erfindungsgemäßer Katalysator | 85 | 57 |
| Vergleichskatalysator 2 | 100 | 61 |

Der erfindungsgemäße Katalysator zeichnet sich aufgrund der definiert eingestellten Aktivkomponentenprofile im Vergleich zum Vergleichskatalysator 1 durch eine signifikant höhere Selektivität zum Wertprodukt Ethylen aus. Zusätzlich wird gegenüber Vergleichskatalysator 2 bei im Wesentlichen gleicher Selektivität ein 90%iger Umsatz schon bei deutlich niedrigerer Temperatur erzielt.

## Patentansprüche

1. Verfahren zur Herstellung eines Schalenkatalysators mit einem Kern und mindestens einer, den Kern umgebenden Schale, wobei
der Kern aus einem inerten Trägermaterial aufgebaut ist,
die mindestens eine Schale aus einer porösen Trägersubstanz aufgebaut ist, wobei die Schale physikalisch an den Kern angeheftet ist, und in der mindestens einen Schale zumindest ein katalytisch aktives Metall, das aus der Gruppe ausgewählt ist, die von den Metallen der 10. und 11. Gruppe des Periodensystems der Elemente gebildet ist, oder eine Vorläuferverbindung des katalytisch aktiven Metalls in gleichmäßig fein verteilter Form enthalten ist, umfassend die Schritte:
a) Bereitstellen eines den Kern des Schalenkatalysator bildenden Trägers aus einem inerten Trägermaterial;
b) Bestreuen des Trägers mit einer oxidischen, pulverförmigen Trägersubstanz, wobei der Träger bewegt wird, unter Ausbildung einer Schale;
c) Besprühen des Trägers mit einem flüssigen Bindemittel, bestehend aus einer wässrigen Lösung einer organischen Verbindung, die bei Normaldruck einen Siedepunkt oder einen Sublimationspunkt von mehr als 100°C aufweist;
d) Einbringen eines katalytisch aktiven Metalls, das ausgewählt ist aus der Gruppe, die gebildet ist von den Metallen der 10. und 11. Gruppe des Periodensystems der Elemente oder einer Vorläuferverbindung dieser Metalle;
e) Verdampfen flüchtiger Bestandteile;
f) gegebenenfalls Aktivieren der Vorläuferverbindung, wobei die Schritte b, c und d gleichzeitig oder in beliebiger Reihenfolge nacheinander sowie gegebenenfalls auch mehrmals ausgeführt werden können,
**dadurch gekennzeichnet, dass** das katalytisch aktive Metall oder die Vorläuferverbindung im Bindemittel gelöst oder suspendiert und während des Besprühens des Trägers mit dem Bindemittel in die Schale des Schalenkatalysators eingebracht oder eine Lösung oder Suspension des katalytisch aktiven Metalls oder der Vorläuferverbindung getrennt auf den Träger aufgesprüht wird, wobei dies während des Aufbaus der Schale oder danach erfolgen kann.

2. Verfahren nach Anspruch 1, **dadurch gekennzeichnet, daß** das Trägermaterial des Kerns porös ist, wobei das Gesamtvolumen der Poren des Trägermaterials bezogen auf das Volumen des Trägermaterials ≤ 1 Vol.-% beträgt.

3. Verfahren nach Anspruch 1 oder 2, **dadurch gekennzeichnet, daß** die Vorläuferverbindung des katalytischen Metalls ein Metalloxid oder ein wasserlösliches Metallsalz ist, vorzugsweise eine Chlorid, ein Nitrat, ein C₁-C₁₀-Carboxylat, ein Carbonat, ein Hydrogencarbonat, ein Sulfat, ein Hydrogensulfat oder ein Phosphat.

4. Verfahren nach Anspruch 1,2 oder 3, **dadurch gekennzeichnet, daß** mehrere, übereinander angeordnete Schalen vorgesehen sind, wobei die Schalen unterschiedliche katalytisch aktive Metalle enthalten können und/oder die Konzentration des katalytisch aktiven Metalls in den benachbarten Schalen unterschiedlich ist.

5. Verfahren nach einem der Ansprüche 1 bis 4, **dadurch gekennzeichnet, daß** die Schichtdicke der Schale zwischen 1 und 1000 µm, vorzugsweise zwischen 10 und 500 µm, insbesondere zwischen 50 und 300 µm beträgt.

6. Verfahren nach einem der Ansprüche 1 bis 5, **dadurch gekennzeichnet, daß** der Träger zuerst mit einer Schicht mit trockener pulverförmiger Trägersubstanz beschichtet wird und anschliessend mit Bindemittel befeuchtet wird, und die Schale durch Bestreuen mit trockenem pulverförmigen Trägermaterial fertiggestellt wird.

7. Verfahren nach einem der Ansprüche 1 bis 5, **dadurch gekennzeichnet, daß** das Bestreuen des Trägers mit pulverförmigem Trägermaterial und das Besprühen des Trägers mit Bindemittel gleichzeitig durch räumlich getrennte Zuführungen erfolgt.

8. Verfahren nach einem der Ansprüche 1 bis 7, **dadurch gekennzeichnet, daß** eine erste Schale auf dem Träger aufgebracht wird, und anschliessend eine weitere Schale aufgebracht wird, die vorzugsweise eine von der ersten Schale verschiedene Zusammensetzung aufweist.

9. Verfahren nach einem der Ansprüche 1 bis 8, **dadurch gekennzeichnet, daß** der Schalenkatalysator nach dem Verdampfen flüchtiger Bestandteile bei einer Temperatur von 200 bis 600 °C calciniert wird.

10. Schalenkatalysator, herstellbar nach dem Verfahren gemäß einem der Ansprüche 1 bis 9.

11. Verwendung des Schalenkatalysators gemäß Anspruch 10 zur Reduktion oder Reinigung von ungesättigten Kohlenwasserstoffen, vorzugsweise von C₂-C₄ Schnitten.

## Claims

1. A process for producing a coated catalyst having a core and at least one shell surrounding the core, where
the core is made up of an inert support material,
the shell or shells is/are made up of a porous support substance, with the shell being attached physically to the core, and at least one catalytically active metal selected from the group consisting of the metals of the 10th and 11th groups of the Periodic Table of the Elements, or a precursor compound of the catalytically active metal, is present in uniformly distributed, finely divided form in the shell or shells, which comprises the steps:
a) providing a support comprising an inert support material to form the core of the coated catalyst;
b) sprinkling the support with an oxidic, pulverulent support substance while keeping the support in motion, to form a shell;
c) spraying the support with a liquid binder comprising an aqueous solution of an organic compound which has a boiling point or sublimation point at atmospheric pressure of more than 100°C;
d) introducing a catalytically active metal selected from the group consisting of the metals of the 10^{th} and 11^{th} groups of the Periodic Table of the Elements or a precursor compound of these metals;
e) evaporating volatile constituents;
f) if appropriate, activating the pressure compound, where steps b, c and d can be carried out simultaneously or in succession in any order and can also, if desired, be carried out two or more times, wherein the catalytically active metal or the precursor compound is dissolved or suspended in the binder and introduced into the shell of the coated catalyst while spraying the support with the binder or a solution or suspension of the catalytically active metal or the precursor compound is sprayed separately onto the support, either during the building up of the shell or afterward.

2. A process as claimed in claim 1, wherein the support material of the core is porous and the total volume of the pores of the support material relative to the volume of the support material is ≤ 1% by volume.

3. A process as claimed in claim 1 or 2, wherein the precursor compound of the catalytic metal is a metal oxide or a water-soluble metal salt, preferably a chloride, a nitrate, a C₁-C₁₀-carboxylate, a carbonate, a hydrogencarbonate, a sulfate, a hydrogensulfate or a phosphate.

4. A process as claimed in any of claims 1, 2 and 3, wherein a plurality of superposed shells are provided, where the shells may comprise different catalytically active metals and/or the concentration of the catalytically active metal is different in adjacent shells.

5. A process as claimed in any of claims 1 to 4, wherein the thickness of the shell is from 1 to 1000 µm, preferably from 10 to 500 µm, in particular from 50 to 300 µm.

6. A process as claimed in any of claims 1 to 5, wherein the support is firstly coated with a layer of dry pulverulent support substance and subsequently moistened with binder, and the shell is produced by sprinkling with dry pulverulent support material.

7. A process as claimed in any of claims 1 to 5, wherein the sprinkling of the support with pulverulent support material and the spraying of the support with binder are carried out simultaneously by means of physically separate feed lines.

8. A process as claimed in any of claims 1 to 7, wherein the first shell is applied to the support and a further shell which preferably has a composition different from that of the first shell is subsequently applied.

9. A process as claimed in any of claims 1 to 8, wherein the coated catalyst is calcined at from 200 to 600°C after evaporation of volatile constituents.

10. A coated catalyst which can be produced by the process as claimed in any of claims 1 to 9.

11. The use of a coated catalyst as claimed in claim 10 for the reduction or purification of unsaturated hydrocarbons, preferably C₂-C₄ fractions.

## Revendications

1. Procédé pour la préparation d'un catalyseur à coquille avec un noyau et au moins une coquille entourant le noyau, le noyau étant élaboré à partir d'un matériau support inerte, ladite au moins une coquille étant élaborée à partir d'une substance support poreuse, la coquille adhérant physiquement au noyau et ladite au moins une coquille contenant au moins un métal catalytiquement actif, choisi dans le groupe formé par les métaux du 10ème et 11ème groupe du système périodique des éléments, ou un composé précurseur du métal catalytiquement actif, sous une forme régulière finement divisée, comprenant les étapes
a) de préparation d'un support formant le noyau du catalyseur à coquille en un matériau support inerte ;
b) de saupoudrage du support avec une substance support oxyde sous forme de poudre, le support étant en mouvement, avec formation d'une coquille ;
c) de pulvérisation sur le support d'un liant liquide, constitué d'une solution aqueuse d'un composé orgarique, qui présente à pression normale un point d'ébullition ou un point de sublimation de plus de 100°C ;
d) d'introduction d'un métal catalytiquement actif, choisi dans le groupe formé par les métaux du 10ème et 11ème groupe du système périodique des éléments ou d'un composé précurseur de ces métaux ;
e) d'évaporation des constituants volatils ;
f) le cas échéant d'activation du composé précurseur, les étapes b, c et d pouvant être réalisées simultanément ou dans un ordre quelconque l'une après l'autre ainsi que le cas échéant aussi plusieurs fois,
**caractérisé en ce que** le métal catalytiquement actif ou le composé précurseur est dissous ou mis en suspension dans le liant et est introduit, pendant la pulvérisation du liant sur le support, dans la coquille du catalyseur à coquille ou **en ce qu'**une solution ou une suspension du métal catalytiquement actif ou d'un composé précurseur est pulvérisée séparément sur le support, ceci pouvant être réalisé pendant l'élaboration de la coquille ou après celle-ci.

2. Procédé selon la revendication 1, **caractérisé en ce que** le matériau support du noyau est poreux, le volume total des pores du matériau support par rapport au volume du matériau support étant ≤ 1 % en volume.

3. Procédé selon la revendication 1 ou 2, **caractérisé en ce que** le composé précurseur du métal catalytique est un oxyde de métal ou un sel de métal soluble dans l'eau, de préférence un chlorure, un nitrate, un carboxylate en C₁ à C₁₀, un carbonate, un hydrogénocarbonate, un sulfate, un hydrogénosulfate ou un phosphate.

4. Procédé selon la revendication 1, 2 ou 3, **caractérisé en ce qu'**on a prévu plusieurs coquilles disposées l'une au-dessus de l'autre, les coquilles pouvant contenir différents métaux catalytiquement actifs et/ou la concentration du métal catalytiquement actif étant différente dans les coquilles adjacentes.

5. Procédé selon l'une quelconque des revendications 1 à 4, **caractérisé en ce que** l'épaisseur de couche des coquilles est comprise entre 1 et 1000 µm, de préférence entre 10 et 500 µm, en particulier entre 50 et 300 µm.

6. Procédé selon l'une quelconque des revendications 1 à 5, **caractérisé en ce que** le support est d'abord revêtu d'une couche de substance support sèche sous forme de poudre et est ensuite humidifié avec l'agent liant et la coquille est terminée par saupoudrage avec du matériau support sec sous forme de poudre.

7. Procédé selon l'une quelconque des revendications 1 à 5, **caractérisé en ce que** le saupoudrage du support avec du matériau support sous forme de poudre et la pulvérisation du support avec l'agent liant sont réalisés simultanément par des alimentations séparées dans l'espace.

8. Procédé selon l'une quelconque des revendications 1 à 7, **caractérisé en ce qu'**on applique une première coquille sur le support, puis une autre coquille, qui présente de préférence une composition différente de celle de la première coquille.

9. Procédé selon l'une quelconque des revendications 1 à 8, **caractérisé en ce que** le catalyseur à coquille est calciné après l'évaporation de constituants volatils à une température de 200 à 600°C.

10. Catalyseur à coquille pouvant être préparé selon le procédé selon l'une quelconque des revendications 1 à 9.

11. Utilisation du catalyseur à coquille selon la revendication 10 pour la réduction ou la purification d'hydrocarbures insaturés, de préférence les fractions en C₂ à C₄.
